Europäisches Patentamt

European Patent Office

Office européen des brevets

(11) Veröffentlichungsnummer: **0 161 551**
**B1**

(12) # EUROPÄISCHE PATENTSCHRIFT

(45) Veröffentlichungstag der Patentschrift:
**25.01.89**

(51) Int. Cl.⁴: **A 61 K 35/14,** B 01 D 17/032

(21) Anmeldenummer: **85105007.0**

(22) Anmeldetag: **25.04.85**

(54) **Vorrichtung zum Entfernen einer Blutplasmaschicht und einer Buffycoatschicht aus einem flexiblen Beutel mit Blutfüllung.**

(30) Priorität: **14.05.84 DE 3417892**

(43) Veröffentlichungstag der Anmeldung:
**21.11.85 Patentblatt 85/47**

(45) Bekanntmachung des Hinweises auf die Patenterteilung:
**25.01.89 Patentblatt 89/4**

(84) Benannte Vertragsstaaten:
**FR GB IT NL SE**

(56) Entgegenhaltungen:
GB-A-2 046 612
US-A-3 545 671

(73) Patentinhaber: **NPBI Nederlands Produktielaboratorium voor Bloedtransfusieapparatuur en Infusievloeistoffen B.V., Runde ZZ41, NL- 7881 HM Emmer Compascuum (NL)**

(72) Erfinder: **Loos, Johannes Antonius, Plesmanlaan 125, NL- 1006 AD Amsterdam (NL)**
Erfinder: **Eitjes, Willem, Plesmanlaan 125, NL- 1006 AD Amsterdam (NL)**
Erfinder: **de Bruyn, Johannes Cornelius Gerardus Hendrikus, Plesmanlaan 125, NL- 1006 AD Amsterdam (NL)**

(74) Vertreter: **Masch, Karl Gerhard, Patentanwälte Andrejewski, Honke & Partner Theaterplatz 3 Postfach 10 02 54, D-4300 Essen 1 (DE)**

## Description

Die Erfindung betrifft eine dem Patentanspruch 1 entsprechende Vorrichtung zum aufeinanderfolgenden Entfernen einer oberen Blutplasmaschicht und einer mittleren Buffycoatschicht aus einem mit einer oberen Schlauchleitung versehenen flexiblen Beutel mit einer durch Zentrifugieren in die beiden Schichten und eine untere rote Zellkonzentratschicht entmischten Blutfüllung. - Unter Buffycoatschicht wird die verhältnismäßig dünne, im wesentlichen die Leukozyten und Thrombozyten aufweisende Zwischenschicht zwischen dem Blutplasma und roten Zellkonzentrat bzw. den Erythrozyten verstanden.

Bei einer bekannten Vorrichtung der genannten Art (US-PS-4 350 585) sind nur die aus einer Gehäusewandung bestehende, die Fläche des Beutels vollständig überdeckende Pressenplatte und die kleinere untere Pressenplatte vorgesehen. Die Buffycoatschicht, die sich nach Herausdrücken der Blutplasmaschicht oberhalb der kleineren Pressenplatte befindet, soll durch weiteres Zusammenfahren der Pressenanordnung und manuelles Drücken auf den freiliegenden oberen Beutelbereich aus dem Beutel harausgedrück bzw. von der unteren roten Zellkonzentratschicht getrennt werden, was in der Praxis jedoch nicht gelingt, weil sich die sehr dünne Buffycoatschicht im Zuge ihres Herausdrückens mit dem roten Zellkonzentrat mehr oder weinger völlig vermischt.

Beim aufeinanderfolgenden manuellen Entfernen von Blutplasma und Buffycoat aus einem Blutbeutel ist es zwar auch schon bekannt [Vox Sanguinis, 39 (1980) 46 - 39, Seiten 48 bis 51], nach Herausdrücken des Blutplasmas unmittelbar unterhalb der Buffycoatschicht eine Operationsklemme auf den Beutel zu klemmen, um die Buffycoatschicht von der roten Zellkonzentratschicht zu trennen. Auch hier hängt aber die einwandfreie Trennung der einzelnen Schichten von der menschlichen Geschicklichkeit ab.

Der Erfingung liegt die Aufgabe zugrunde, eine Vorrichtung der eingangs genannten Art so weiter auszubilden, daß mit ihr Blutplasma, Buffycoat und rotes Zellkonzentrat einwandfrei und reproduzierbar voneinander getrennt werden können.

Die erfindungsgemäße Lösung dieser Aufgabe ergibt aus dem Kennzeichen des Patentanspruches 1. - Die Erfindung nutzt hierbei die Erkenntnis, daß die bekannte Vorrichtung der eingangs genannten Art von menschlichen Unzulängli...keiten unabhängig gemacht werden kann, indem die anderweitig bekannte Maßnahme des Abklemmens der Buffycoatschicht vorrichtungsmäßig transponiert in diese Vorrichtung integriert wird und zusätzlich eine obere Presse bzw. Pressenplatte zum Herausdrücken der Buffycoatschicht vorgesehen wird.

Für die weitere Ausgestaltung der Erfindung bestehen mehrere Möglichkeiten, deren bevorzugte sich aus den weiteren Patentansprüchen ergeben. Von besonderer Bedeutung sind hierbei zunächst die Patentansprüche 4 und 5, deren Maßnahmen es nämlich erlauben, die Vorrichtung auf einfachste Weise unterschiedlich gestalteten Blutbeuteln anzupassen. Besonders hervorzuheben sind aber auch die Patentansprüche 10 und 11; der Einsatz von Infrarotlicht einer Wellenlänge von 940 nm und einer Frequenz von 68 Hz zum Erfühlen der Blutplasma/Buffycoat-Grenzfläche führt nämlich zu einen besonders zuverlässigen Arbeiten der Vorrichtung, und zwar unabhängig von irgendwelchen Unregelmässikeiten des Tageslichtes bzw. der Raumbeleuchtung und Beutelaufdrucken.

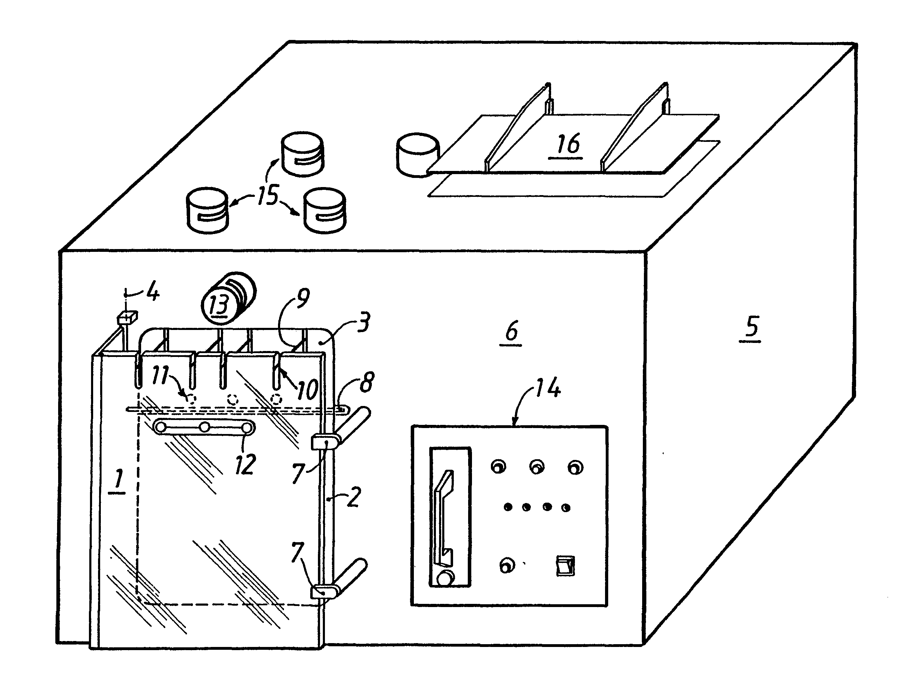

Im folgenden wird die Erfindung anhand einer ein Ausführungsbeispiel darstellenden Zeichnung mit einer einzigen Figur nährer erläutert.

Die in der Figur perspektivisch dargestellte Vorrichtung dient zum aufeinanderfolgenden Entfernen einer oberen Blutplasmaschicht und einer mittleren Buffycoatschicht aus einem flexiblen Beutel aus transparentem Kunststoffmaterial, der mit einer oberen Schlauchleitung versehen ist und eine Blutfüllung mit Antikoagulans aufweist. Die Blutfüllung ist durch Zentrifugieren des gefüllten Beutels in eine obere Blutplasmaschicht, einen mittlere Buffycoatschicht und eine untere rote Zellkonzentratschicht entmischt worden. In ihrem grundsätzlichen Aufbau besteht die Vorrichtung aus einer Pressenanordnung mit vertikalen Pressenplatten 1 - 3, zwischen die der zentrifugierte Beutel einführbar ist und die anschließend in Parallelstellung relativ zusammenfahrbar sind.

Die Pressanordnung besteht auf einer Seite aus einer Pressenplatte 1, welche die Fläche des Beutels vollständig überdeckt und gleichsam als Tür aus einem transparenten Material gefertigt bzw. um eine vertikale Achse 4 verschwenkbar an einem Gehäuse 5 befestigt ist. Nach Einführen eines Beutels wird diese Pressenplatte 1 in eine Stellung mit Abstand von und parallel zur zugeordneten Gehäusefläche 6 gebracht und mit Hilfe von hintergreifenden Riegeln 7 in dieser Stellung gehalten.

Der genannten Pressenplatte 1 gegenüberliegend sind zunächst zwei Pressenplatten 2, 3 vorgesehen, die in ihrer Ausgangsstellung bündig zur zugeordneten Gahäusefläche 6 liegen. Die untere Pressenplatte 2 umfaßt zumindest die der unteren roten Zellkonzentratschicht zugeordnete Fläche des Beutels, die obere Pressenplatte 3 dagegen die restliche Fläche des Beutels. Zwischen der unteren und der oberen Pressenplatte 2 bzw. 3 ist ein Beutelabquetschelement 8 vorgesehen, das aus einer horizontal beweglichen Horizontalplatte besteht und mit dessen Hilfe im Beutel durch Klemmen gleichsam zwei Kammern gebildet werden könne. Im Gehäuse 5 sind die beiden Pressenplatten 2, 3 und das Beutelabquetschelement 8 an voneinander unabhängige Antriebe, z. B. in Form doppeltwirkender Hydraulikzylinderanordnungen, angeschlossen. Im einzelnen nich dargestellt sind die bevorzugten Maßnahmen, nämlich daß die obere Pressenplatte 3 mit einem elastischen Polster belegt ist, welches alle Beutelunebenheiten auffängt und folglich ein vollständiges Ausdrücken des zugeordneten Beutelteils sicherstellt, und daß dem Beutelabquetschelement 8 an der gegenüberliegenden Pressenplatte ein elastisches Widerlager zugeordnet ist, welches Beutelbeschädigungen vermeidet.

In Höhe der oberen Pressenplatte 3 sind Aufhängestifte 9 für den zentrifugierten Beutel vorgesehen. Diese Aufhängestifte 9 kommen paarweise zum Einsatz, da es unterschiedliche Beutelkonstruktionen gibt. Jedenfalls sind die Aufhängestifte 9 für entsprechende Anpassung höhenverstellbar, zu welchem Zweck die obere Pressenplatte 3 und die beim Pressen festgehaltene Pressenplatte 1 mit entsprechenden Vertikalschlitzen 10 versehen sind.

Das Beutelabquetschelement 8 ist mit Hilfe einer Fühleranordnung für die Blutplasma/Buffycoat-Grenfläche steuerbar. Diese Fühleranordnung besteht aus einer höhenmäßig im Bereich der oberen Pressenplatte 3 angeordneten Horizontalreihe von Infrarotlichtquellen 11 und -detektoren, die mit pulsierendem Licht einer Wellenlänge von 940 nm und einer Frequenz von 68 Hz arbeiten. Mit diesen Arbeitsparametern ist eine gegenüber Tageslichtänderungen und überraschenderweise auch eventuellen Beutelbeschriftungen unempfindliches Arbeiten gewährleistet. Ein Schaltimpuls wird nur ausgelöst, wenn bei allen drei Paaren Lichtquelle/ Lichdetektor 11, 12 gleichzeitig das Lich um 80 % für mindestens 0,1 sec. geschwächt wird. Um die Buffycoatschicht höhenmäßig auseinanderzuziehen, ist die obere Pressenplatte 3 vor Betätigung der unteren Pressenplatte 2 mit einem einstellbaren Pressenteilhub vorbewegbar. Durch entsprechende Einstellung dieses Preßteilhubes und der Höhe der Aufhängestifte 9 läßt sich dann für praktisch alle üblichen Blutbeutel erreichen, daß das Beutelabquetschelement 8 den Beutelinhalt stets unmittelbar unterhalb der Buffycoat/Zellkonzentrat-Grenzfläche horizontal unterteilt. Bei der entsprechenden Vorbewegung des Beutelabquetschelementes 8 ist die untere Pressenplatte 2 dann entlastbar, damit das Beutelabquetschelement 8 die Trennung so schnell wie möglich vollzieht.

Im übrigen erkennt man an der Figur, daß oberhalb der Pressenanordnung noch eine Schlauchklemme 13 und neben der Pressenanordnung ein Bedienungsfeld 14 auf der Oberseite des Gehäuses 5 Klemm- sowie Verschweißköpfe 15 für Schläuche und eine Zusatzpresse 16 für Hilfebeutel angeordnet sind.

Die beschriebene Vorrichtung arbeitet folgendermaßen: Der zentrifugierte Blutbeutel wird bei geöffneter Pressenanordnung in die höhenmäßig entsprechend eingestellten Aufhängestifte 9 gehängt und mit seiner Schlauchleitung in die oberhalb der Pressenanordnung befindliche Schlauchklemme 13 eingelegt. Das freie Ende der Schlauchleitung wird über eine Verzweigung mit den in die Klemm- und Verschweißköpfe 15 eingelegten Schlauchleitungen zweier leerer Beutel verbunden, woraufhin die Pressenanordnung durch Verschwenken der vorderen Pressenplatte 1 und Verriegelung derselben über die Riegel 7 geschlossen wird. Die Schlauchklemme 13 und der Klemm- und Verschweißkopf 15 mit der Schlauchleitung für den Beutel, der das Blutplasma aufnehmen soll, werden geöffnet, während der Klemm- und Verschweißkopf 15 mit der Schlauchleitung für den Beutel, der die Buffycoatschicht aufnehmen soll, geschlossen wird. Die obere Pressenplatte 3 fährt nun mit dem vorgegebenen Preßteilhub aus, wobei ein Teil der Blutplasmaschicht in den gewünschten anderen Beutel gedrückt wird. Unmittelbar anschliessend fährt die untere Pressenplatte 2 aus, wobei weiteres Blutplasma abfließt und die Buffycoatschicht steigt. Sobald die Grenzfläche zwischen der Blutplasmaschicht und der Buffycoatschicht von den Infrarotlichtquellen 11 und -detektoren 12 registriert worden ist, wird die Schlauchklemme 13 oberhalb der Pressenanordnung geschlossen, fährt das Beutelabquetschelement 8 aus und wird die untere Pressenplatte 2 entlastet. Auf diese Weise wird die Buffycoatschicht von der roten Zellkonzentratschicht getrennt. Zwischenzeitlich verschließt und verschweißt der Klemm- und Verschweißkopf 15 die Schlauchleitung mit dem Beutel, welcher das Blutplasma enthält, während der Klemm- un Verschweißkopf 15 mit der Schlauchleitung für den Beutel, der für die Buffycoatschicht bestimmt ist, geöffnet wird. Anschließend drückt die obere Pressenplatte 3 die Buffycoatschicht vollständig in den genannten weiteren Beutel. Gewünschtenfalls kann dann anschließend noch mit Hilfe der Zusatzpresse 16 eine Hilfsflüssigkeit (SAGM) der roten Zellkonzentratschicht zugeführt werden. Der beschriebene Ablauf erfolgt jedenfalls selbsttätig.

Die untere Pressenplatte 2 und die obere Pressenplatte 3 müssen nicht notwendigerweise starr ausgeführt bzw. als Ganzes horizontal verstellbar sein. Es besteht nämlich ohne weiteres auch die Möglichkeit, die untere und obere Pressenplatte 2 bzw. 3 jeweils als hydraulisch oder pneumatisch beaufschlagbare Membran auszuführen. Eine solche Ausführungsform ist ebenfalls Gegenstand der Erfindung.

## Patentansprüche

1. Vorrichtung zum aufeinanderfolgenden Entfernen einer oberen Blutplasmaschicht und einer mittleren Buffycoatschicht aus einem mit einer oberen Schlauchleitung versehenen flexiblen Beutel mit einer durch Zentrifugieren in die beiden genannten Schichten und eine untere rote Zellkonzentratschicht entmischten Blutfüllung, bestehend aus einer Pressenanordnung mit vertikalen Pressenplatten, zwischen die der zentrifugierte Beutel einführbar ist und die anschließend in Parallelstellung relativ zusammenfahrbar sind, wobei eine der Pressenplatten festgehalten ist und die Fläche des Beutels vollständig überdeckt und eine dieser gegenüberliegende kleinere untere Pressenplatte zumindest die der unteren roten Zellkonzentratschicht zugeordnete Fläche des Beutels umfaßt, dadurch gekennzeichnet, daß oberhalb der unteren Pressenplatte (2) einen unabhängig von dieser betätigbare, der restlichen Fläche des Beutels zugeordnete obere Pressenplatte (3) vorgesehen ist und daß zwischen der unteren sowie oberen Pressenplatte (2, 3) ein horizontal bewegliches Abquetschelement (8) angeordnet ist.

2. Vorrichtung nach Anspruch 1, dadurch gekennzeichnet, daß die untere sowie die obere Pressenplatte (2, 3) und das Beutelabquetschelement (8) an einer Seite eines zugeordnete Antriebe aufnehmenden Gehäuses (5) vorgesegen sind und die die gesamte Fläche des Beutels überdeckende Pressenplatte (1) um eine vertikale Achse (4) verschwenkbar am Gehäuse (5) befestigt sowie mit Hilfe von Riegeln (7) in einem vorgegebenen Abstand vom Gehäuse (5) haltbar ist.

3. Vorrichtung nach Anspruch 1 oder 2, dadurch gekennzeichnet, daß die obere Pressenplatte (3) mit einem elastischen Polster belegt ist.

4. Vorrichtung nach einem der Ansprüche 1 bis 3, dadurch gekennzeichnet, daß die obere Pressenplatte (3) vor Betätigung der unteren Pressenplatte (2) mit einem einstellbaren Preßteilhub vorbewegbar ist.

5. Vorrichtung nach einem der Ansprüche 1 bis 4, gekennzeichnet durch höhenverstellbare Aufhängestifte (9) für den zentrifugierten Beutel.

6. Vorrichtung nach einem der Ansprüche 1 bis 5, dadurch gekennzeichnet, daß dem Beutelabquetschelement an der gegenüberliegenden Pressenplatte (1) ein elastisches Widerlager zugeordnet ist.

7. Vorrichtung nach einem der Ansprüche 1 bis 6, dadurch gekennzeichnet, daß die untere Pressenplatte (2) bei Vorbewegung des Beutelabquetschelementes (8) entlastbar ist.

8. Vorrichtung nach einem der Ansprüche 1 bis 7, dadurch gekennzeichnet, daß die Vorbewegung des Beutelabquetschelementes (8) mit Hilfe einer Fühleranordnung für die Blutplasma/Buffycoat-Grenzfläche steuerbar ist.

9. Vorrichtung nach einem der Ansprüche 1 bis 8, dadurch gekennzeichnet, daß die Fühleranordnung aus einer höhenmäßig im Bereich der oberen Pressenplatte (3) angeordneten Horizontalreihe von Infrarotlichtquellen (11) und -detektoren (12) besteht.

10. Vorrichtung nach Anspruch 9, dadurch gekennzeichnet, daß die Infrarotlichtquellen (11) und -detektoren (12) für pulsierendes Licht einer Wellenlänge von 940 nm ausgelegt sind.

11. Vorrichtung nach Anspruch 10, dadurch gekennzeichnet, daß die Infrarotlichtquellen (11) und -detektoren (12) für Licht einer Frequenz von 68 Hz ausgelegt sind.

## Claims

1. Equipment for the successive removal of an upper blood plasma layer and an intermediate Buffycoat layer from a flexible bag provided with an upper flexible tube and filled with blood, separated by centrifuging into the two abovenamed layers and a lower red corpuscle concentrate layer, consisting of a press device with vertical press plates between which the centrifuged bag is insertable and which is subsequently movable relatively together in a parallel position, in which one of the press plates is held stationary and completely overlaps the surface of the bag, and a smaller lower press plate opposite to it encompasses at least the area of the bag corresponding to the lower red corpuscle concentrate layer, characterized in that above the lower press plate (2) and operable independently thereof is an upper press plate (3) corresponding to the remaining area of the bag, and that a horizontally-movable squeezing unit (8) is located between the lower and the upper press plates (2, 3).

2. Equipment according to Claim 1, characterized in that the lower and the upper press plates (2, 3) and the bag-squeezing unit (8) are provided on one side of a housing (5) that incorporates associated drives, and the press plate (1) that overlaps the whole area of the bag is attached to housing (5), is swingable about a vertical axis (4), and holdable at a specified distance from housing (5), by means of lock bolts (7).

3. Equipment according to Claim 1 or 2, characterized in that the upper press plate (3) is covered by an elastic cushion.

4. Equipment according to Claims 1 to 3, characterized in that the upper plate (3) is movable forward by an adjustable partial press stroke before actuation of the lower press plate (2).

5. Equipment according to one of Claims 1 to 4, characterized by suspension pins (9) adjustable in height, for the cetrifuged bag.

6. Equipment according to one of Claims 1 to 5, characterized in that on the opposite press plate (1) an elastic buttress is associated with the bag-squeezing unit.

7. Equipment according to one of Claims 1 to 6, characterized in that the lower press plate (2) is releasable by movement forwards of the bag squeezing unit (8).

8. Equipment according to one of Claims 1 to 7, characterized in that the forward movement of the bag squeezing unit (8) is controllable by means of a sensor device for the blood plasma/Buffycoat boundary surface.

9. Equipment according to one of Claims 1 to 8, characterized in that the sensor device consists of a horizontal row of infra-red light sources (11) and detectors (12) located at a level in the area of the upper press plate (3).

10. Equipment according to Claim 9, characterized in that the infra-red light sources (11) and detectors (12) are designed for pulsating light of a wavelength of 94nm.

11. Equipment according to Claim 10, characterized in that the infra-red light sources (11) and detectors (12) are designed for light of a frequency of 68 Hz.

**Revendications**

1. Dispositif pour l'élimination successive d'une couche supérieure de plasma sanguin et d'une couche intermédiaire de couenne inflammatoire d'un sachet souple muni d'un tuyau souple supérieur et rempli de sang dissocié par centrifugation en lesdites deux couches et en une couche inférieure de concentré d'hématies, comprenant un système de presse avec des plateaux de presse verticaux entre lesquels peut être inséré le sachet centrifugé et qui peuvent ensuite être rapprochés les uns des autres en position parallèle, l'un des plateaux de presse étant maintenu en place et recouvrant entièrement la surface du sachet et un plateau de presse inférieur de dimensions plus petites situé en face du plateau de presse précité recouvrant au moins la surface du sachet associée à la couche inférieure de concentré d'hématies, caractérisé par le fait que, au-dessus du plateau de presse inférieur (2) est prévu un plateau de presse supérieur (3) associé à la surface restante du sachet et pouvant être actionné indépendamment dudit plateau inférieur, et qu'un élément de pincement (8) mobile dans le sens horizontal est disposé entre les plateaux de presse inférieur et supérieur (2, 3).

2. Dispositif selon la revendication 1, caractérisé par le fait que les plateaux de presse inférieur et supérieur (2, 3) et l'élément de pincement de sachet (8) sont prévus sur un côté d'un boîtier (5) abritant des mécanismes d'entraînement associés, et que le plateau de presse (1) recouvrant la totalité de la surface du sachet est monté sur le boîtier (5) de façon à pouvoir pivoter autour d'un axe vertical (4) et pouvant être maintenu, au moyen de verrous (7), à une distance prédéterminée du boîtier (5).

3. Dispositif selon l'une des revendications 1 ou 2, caractérisé par le fait que le plateau de presse supérieur (3) est garni d'un matelas élastique.

4. Dispositif selon l'une quelconque des revendications 1 à 3, caractérisé par le fait que le plateau de presse supérieur (3) peut effectuer, avant l'actionnement du plateau de presse inférieur (2), une course partielle réglable de la presse.

5. Dispositif selon l'une quelconque des revendications 1 à 4, caractérisé par le fait qu'il comprend des broches de suspension (9) réglables en hauteur pour le sachet centrifugé.

6. Dispositif selon l'une quelconque des revendications 1 à 5, caractérisé par le fait qu'à l'élément de pincement de sachet est associée une butée élastique sur le plateau de presse opposé (1).

7. Dispositif selon l'une quelconque des revendications 1 à 6, caractérisé par le fait que le plateau de presse inférieur (2) peut être déchargé lors de l'avancement de l'élément de pincement de sachet (8).

8. Dispositif selon l'une quelconque des revendications 1 à 7, caractérisé par le fait que l'avancement de l'élément de pincement de sachet (8) peut être commandé à l'aide d'un système de capteurs pour la surface limite de plasma sanguin/couenne inflammatoire.

9. Dispositif selon l'une quelconque des revendications 1 à 8, caractérisé par le fait que le système de capteurs est constitué d'une rangée horizontale de sources de lumière infrarouge (11) et de détecteurs de lumière infrarouge (12) disposés en hauteur dans la région du plateau de presse supérieur (3).

10. Dispositif selon la revendication 9, caractérisé par le fait que des sources de lumière infrarouge (11) et des détecteurs de lumière infrarouge (12) sont conçus pour de la lumière pulsée d'une longueur d'onde de 940 nm.

11. Dispositif selon la revendication 10, caractérisé par le fait que les sources de lumière infrarouge (11) et les détecteurs de lumière infrarouge (12) sont conçus pour de la lumière d'une fréquence de 68 Hz.